# EUROPEAN PATENT APPLICATION

(11) **EP 4 335 858 A1**
(43) Date of publication of application: **13.03.2024**
(21) Application number: 22799011.6
(22) Date of filing: 11.04.2022
(51) Int. Cl.: C07H 15/10, C07H 1/00, C12P 19/44, C07B 63/00

(54) **METHOD FOR PURIFYING SOPHOROLIPID**

(30) Priority: 03.05.2021 KR 20210057233
(71) Applicant: Macrocare Tech., Ltd., Cheongju-si, Chungcheongbuk-do 28126 (KR)
(72) Inventor: KIM, Moo Sung, Suwon-si Gyeonggi-do 16527 (KR); LEE, Sang Rin, Seoul 04959 (KR)
(74) Representative: Modiano, Gabriella Diana
(86) International application number: PCT/KR2022/005170
(87) International publication number: WO 2022/234966

(57) **Abstract**

The present disclosure relates to a method of separating and purifying sophorolipids having improved physical properties such as unusual odor, color, and viscosity from oxidized and lactonic sophorolipids precipitate produced by culturing the genus Candida yeast fungus.

## Description

### BACKGROUND

### 1. Field of the Invention

The present disclosure relates to a method of separating and purifying sophorolipids having improved physical properties such as unusual odor, color, and viscosity from oxidized and lactonic sophorolipids precipitate produced by culturing the genus Candida yeast fungus.

### 2. Discussion of Related Art

Surfactants are compounds that have both hydrophilic and hydrophobic groups within one monomer molecule, and have the function of lowering the interfacial tension by adsorbing at the interface between two or more substances. Surfactants may be broadly classified into ionic surfactants, which exhibit ions in an aqueous solution, and nonionic surfactants, which do not ionize, and other classification criteria include chemical surfactants produced by chemical synthesis and biosurfactants produced by microorganisms such as bacteria, yeast, and mold. Among them, biosurfactants show low toxicity to the human body and are easily biodegraded due to their simple structure and constituent substances. In addition, when the surfactant exceeds a certain concentration in an aqueous solution, the interfacial tension is maintained without further decrease, and at the same time, exhibits the characteristic of forming micelles. The concentration that forms micelles at this time is called critical micelle concentration (CMC), and because biosurfactants have a lower CMC compared to surfactants made by chemical synthesis, they show better cleaning power than chemical surfactants, and much research is being performed as a replacement for chemical surfactants.

Biosurfactants refer to all types of surfactants produced through the biosynthetic metabolic pathways of microorganisms such as bacteria, yeast, and mold, and are classified according to its structure into glycolipids, lipopeptides and lipoproteins, neutral fats and phospholipids, polymer surfactants, particle surfactants, and the like. Sophorolipid has has a simple structure in which sophorose which is a β-1,2 bond of two glucose molecules and a fatty acid with a hydroxyl group, especially one unsaturated fatty acid with a 16 or 18 carbon chain, are bonded, and has high cleaning power and shows high antibacterial activity against bacteria and fungi, so that the sophorolipid is used in various fields such as cosmetics and food.

The structure of sophorolipids produced from yeast fungus may be broadly divided into two types: oxidized (acidic) sophorolipids and closed type lactonic sophorolipids, and their antibacterial activity and physical properties differ depending on their structures. According to current research, sophorolipids are mainly produced through yeast fungus culture, and among them, strains of the genus Candida are known to show high production concentrations, and because the *Candida bombicola* strain shows particularly high productivity, much research is being done on the strain and it is being used in production research [Kurtzman et al, FEMS microbiology letters, 311(2), 140-146. (2010)].

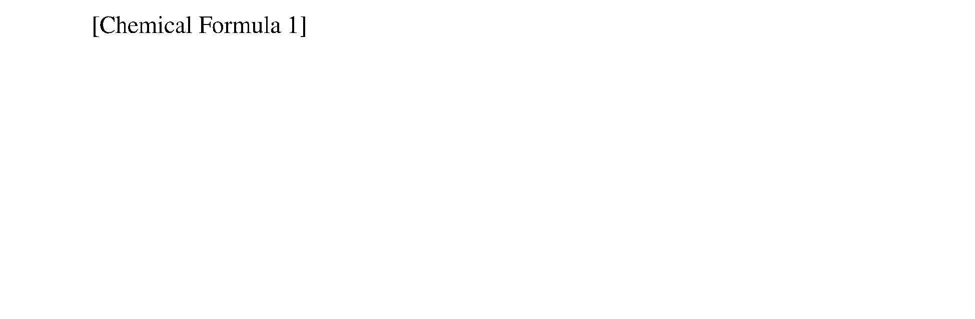

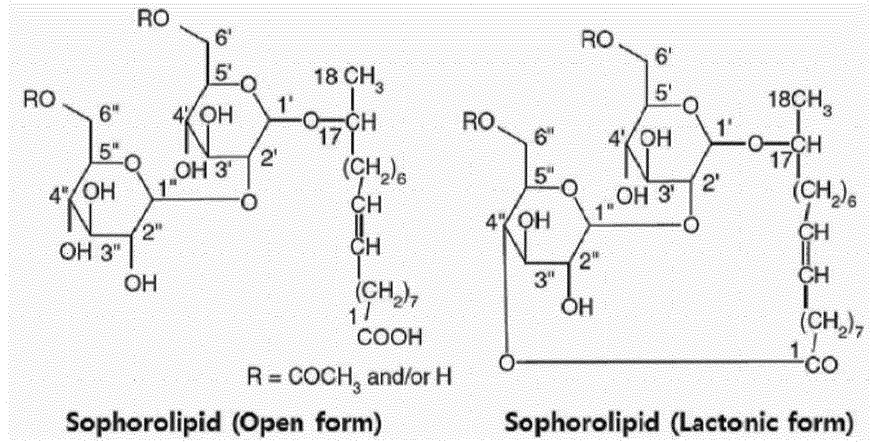

When performing fed-batch culture to maintain a constant concentration of glucose, which is a carbon source, and oil, which is an unsaturated fatty acid source, among the genus Candida yeast fungus, *Candida bombicola* is known to exhibit a sophorolipid production concentration of up to 300 g/L, and in general, in the case of sophorolipids produced through culture of *Candida bombicola,* oxidized and lactone forms account for 30-40% and 60-70%, respectively. The lactonic sophoroipids showed lower CMC than the oxidized sophoroipids and had better cleaning power. While the oxidized sophoroipids showed little antibacterial activity against bacteria, the lactonic sophoroipids showed antibacterial activity against bacteria such as Propionibacterium acnes, which is an acne bacteria. The oxidized form has a higher HLB (Hydrophilic Lipophilic Balance) value than the lactonic form, which indicates that the oxidized form has high solubility in water and may act as a solubilizer as a hydrophilic surfactant.

Methods for separating and purifying surfactants with a glycolipid structure, including sophorolipids, which are biological surfactants showing functionalities such as cleaning power and antibacterial activity, include a extraction method using organic solvents, and a crystallization method using a buffer as a representative example, and depending on the substance used for purification, the ratio and composition of a mixture of the oxidized and lactonic sophorolipids vary, and also affect purity and yield. Among the above purification methods, hexane and ethyl acetic acid are mainly used for purification using the organic solvent, and since the use of the organic solvent is toxic to the human body, there are restrictions on its use when the residual solvent is not removed, and the purity of the purification is also low. The crystallization method using the buffer may separate sophorolipids of a specific structure among the oxidized and lactonic sophorolipids with high purity, but the disadvantage is that the yield is low. In addition, there is a purification method using a column, but because recovery of sophorolipids is difficult, and organic solvents are used, problems may arise in application to cosmetics, etc., as with the extraction method using the organic solvent [Saraya Co., Ltd. (2015). E. P. Patent No. 2821495A1].

Moreover, the mixture of the oxidized and lactonic sophorolipids purified through extraction using the organic solvent has a high viscosity due to the nature of the substance, so that it is not easy to handle in the cosmetic formulation, and the unusual odor and turbid color make it difficult to apply in various fields such as household goods such as detergents, including cosmetics. In the case of the unusual odor, it can be improved by removing an acetyl group of a hydrophilic group of the sophorolipid, but, for this purpose, there are methods using strong base treatment or enzymes, and these have the disadvantage of decomposing the sophorolipid or reducing its conversion efficiency.

### SUMMARY OF THE INVENTION

The present disclosure is for the purpose of providing a method of purifying sophorolipids with improved unusual odor, color, and viscosity through an environmentally friendly method that does not use an organic solvent.

### [Means to solve the problem]

In order to achieve the above matter, the present disclosure provides a method of purifying sophorolipids including:
(1) ultrafiltering a sophorolipid precipitate from a culture fluid of the genus Candida yeast fungus;
(2) adding ethanol and activated carbon to the ultrafiltration residue, adsorbing it, and then removing the activated carbon and ethanol; and
(3) mixing the solution obtained in operation (2) with water to prepare an aqueous solution, and adjusting the pH of the aqueous solution to pH 5 or more and less than pH 7.

### [Advantageous Effects]

According to the present disclosure, it is possible to obtain sophorolipids with improved physical properties such as unusual odor, odor, color, and fluidity through an eco-friendly method that does not use an organic solvent, so that it can be applied to various fields including cosmetic formulations.

### [Brief Description of Drawings]

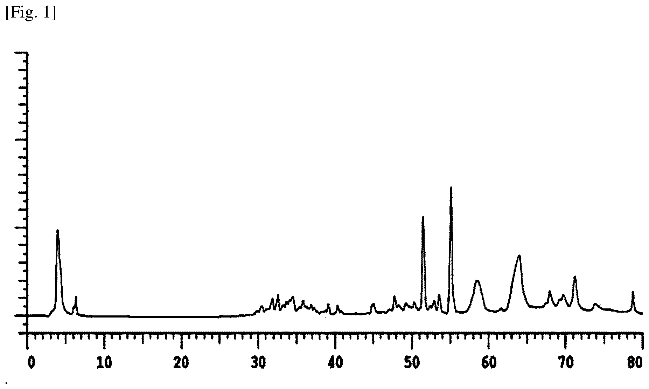

FIG. 1 is a graph showing the results of analysis of an oxidized sophorolipids and a lactonic sophorolipids using high-performance liquid chromatography (HPLC) to identify the sophorolipids separated after filtering the sophorolipid precipitate produced by culturing the genus Candida yeast fungus using an ultrafiltration membrane having a molecular weight cutoff of 10,000 Daltons.

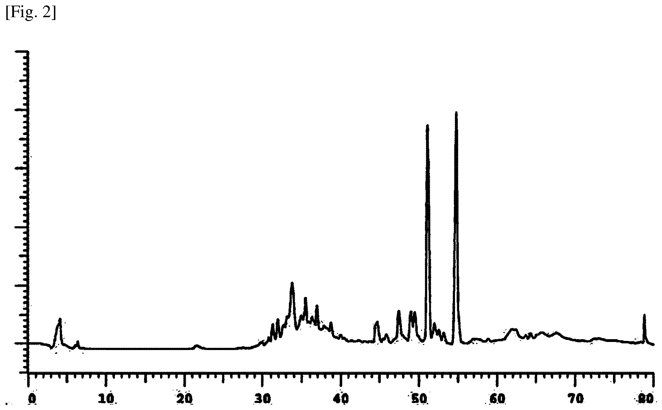

FIG. 2 is a graph showing the results of analysis of an oxidized sophorolipids and a lactonic sophorolipids using high-performance liquid chromatography (HPLC) to identify the sophorolipids after filtering the sophorolipid precipitate produced by culturing the genus Candida yeast fungus using an ultrafiltration membrane having a molecular weight cutoff of 10,000 Da and separating it using an ultrafiltration membrane having a molecular weight cutoff of 3,000 Da.

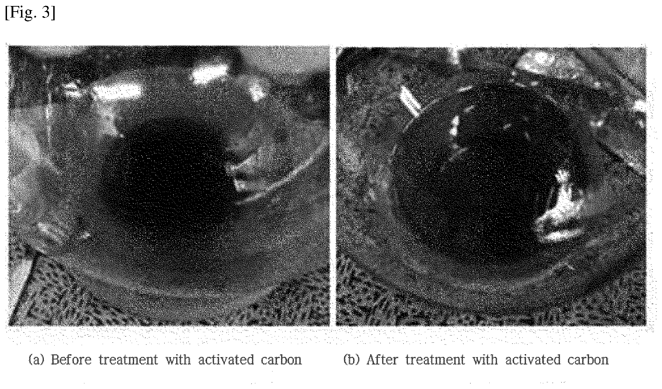

FIG. 3 is a photograph showing the appearance of the sophorolipids before and after the process of removing activated carbon and ethanol after adding and adsorbing the activated carbon and ethanol to the mixture of the oxidized and lactonic sophorolipids treated with ultrafiltration.

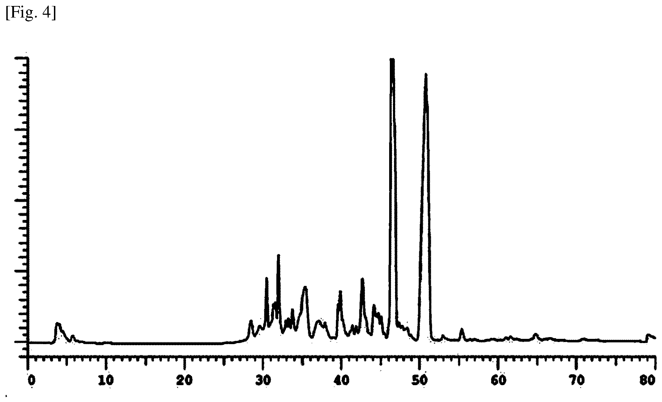

FIG. 4 is a graph showing the results of analysis of an oxidized sophoroipids and a lactonic sophoroipids using high-performance liquid chromatography (HPLC) to identify the sophorolipids after removing the activated carbon and ethanol after adding and adsorbing activated carbon and ethanol to the mixture of the oxidized and lactonic sophorolipids treated with ultrafiltration.

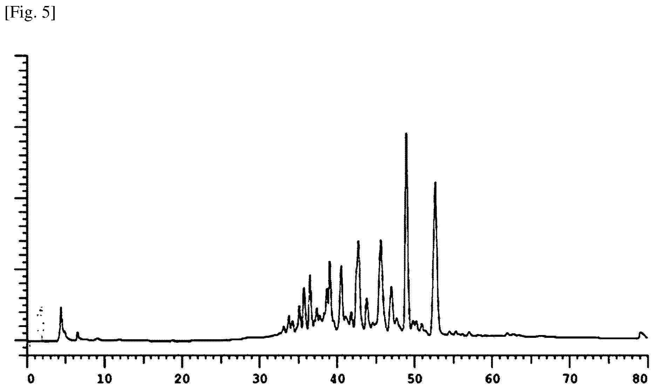

FIG. 5 is a graph showing the results of analysis of an oxidized sophoroipids and a lactonic sophoroipids using high-performance liquid chromatography (HPLC) to identify the sophorolipids after removing activated carbon and ethanol, mixing water and then adjusting the pH of the aqueous solution.

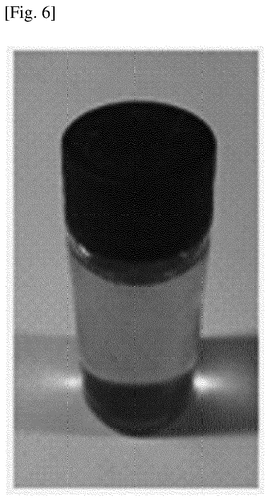

FIG. 6 is a photograph observing the appearance of sophoropyrides purified through a purification method according to an embodiment of the present disclosure.

### DETAILED DESCRIPTION OF EXEMPLARY EMBODIMENTS

Hereinafter, the present disclosure will be described in more detail.

A method of preparing sophorolipids of the present disclosure includes:
(1) ultrafiltering a sophorolipid precipitate from a culture fluid of the genus Candida yeast fungus;
(2) adding ethanol and activated carbon to the ultrafiltration residue, adsorbing it, and then removing the activated carbon and ethanol; and
(3) mixing the solution obtained after removing the activated carbon with water to prepare an aqueous solution, and adjusting the pH of the aqueous solution to pH 5 or more and less than pH 7.

Each operation is described in detail below.

### (1) Ultrafiltering a sophorolipid precipitate from a culture fluid of the genus Candida yeast fungus.

First, the sophorolipid precipitate produced from a culture fluid of the genus Candida yeast fungus is recovered, and the oxidized and lactonic sophorolipids are separated and purified using membrane filtration. The membrane filtration is a method of separating and purifying substances based on the pore size using a membrane having a specific pore size, using the difference in molecular weight between the substance to be purified and the impurities to be removed.

Specifically, the present disclosure separates and purifies the oxidized and lactonic sophorolipids from sophorolipid precipitates using ultrafiltration. This is due to the amphipathic structure among the structural properties of surfactants, that is, the characteristic of having both hydrophilic and hydrophobic groups in one molecule, and surfactants form a micelle structure at a specific concentration depending on pH or temperature in an organic solvent or aqueous solution. The surfactant adsorbs to the interface and lowers the interfacial tension, and when the concentration exceeds a certain concentration, the interfacial tension is no longer reduced and is maintained at that level, and the concentration is set as the critical micelle concentration (CMC), and micelles are formed at concentrations above the CMC. A micelle is an aggregate of surfactant monomers gathered together.

In an aqueous solution, the hydrophilic functional group of the surfactant faces the outside of the sphere, and the hydrophobic functional group has a structure that forms a nucleus in the center, which structure is called a micelle. The oxidized and lactonic sophophoretic lipids have CMC values of 0.1 wt% and 0.01 wt%, respectively, under standard conditions, and sophorolipid monomers other than dissolved sophorolipids accumulate above that concentration to form micelles. When a surfactant forms micelles, the number of surfactant monomers in the aggregate varies depending on the structure, temperature, concentration, and the like of the substance, which may be expressed as aggregation number (NAgg). In the literature, the aggregation number of sophorolipids is 6 (that is, when sophorolipids form micelles, six monomers form one micelle). Since the monomer molecular weights of the oxidized and lactonic sophorolipids are 706 g/mol and 688 g/mol, respectively, and sophorolipids have a micelle structure and have an average molecular weight of 4240 g per mole for the oxidized form and 4130 g for the lactonic form in aqueous solution.

Based on the molecular weight when forming the micelle structure of the sophorolipid, the molecular weight of the culture fluid components, proteins, and metabolites to be removed were confirmed in order to select the pore size of the filtration membrane suitable for membrane filtration according to molecular weight. Among the components in the culture fluid, glucose, which a carbon source, is 180 g/mol, oils including unsaturated fatty acids are 280 g/mol, and pigment components are about 400 g/mol, most of which are less than 1000 g/mol. In the case of proteins, since the molecular weight distribution is wide, ultrafiltration is necessary to remove proteins with relatively large molecular weights of 10,000 Da or more. Accordingly, the ultrafiltration of the present disclosure includes filtration with an ultrafiltration membrane having a molecular weight cutoff of 10,000 Da to remove impurities such as glucose, oil-including culture fluid components, proteins, and metabolites, and filtration with an ultrafiltration membrane having a molecular weight cutoff of 1,000 to 3,000 Da to separate impurities with a molecular weight of 1,000 to 3,000 Da or less.

In the ultrafiltration, a large molecule such as protein is removed through filtration with an ultrafiltration membrane having a molecular weight cutoff of 10,000 Da, and then filtered with an ultrafiltration membrane having a molecular weight cutoff of 1,000 to 3,000 Da to separate impurities with a molecular weight of 1,000 to 3,000 Da or less. At this time, sophorolipids that are dispersed without dissolving in water and sophorolipids that form micelles do not pass through the membrane and are concentrated outside the filtration membrane. A mixture of the oxidized and lactonic sophorolipids concentrated through membrane filtration is recovered, concentrated in vacuum, and dried.

### (2) Next, adding ethanol and activated carbon to the ultrafiltration residue, adsorbing it, and then removing the activated carbon and ethanol.

This is a process to remove highly turbid brown color and abnormal odor-causing substances through treatment with activated carbon on a mixture of the oxidized and lactonic sophorolipids separated and purified through membrane filtration. Activated carbon is a porous material including carbon as its main component, and has the ability to adsorb various substances depending on its structure and characteristics. The activated carbon is made from plants, coal, and the like, activated through acid treatment using steam or phosphoric acid, and classified into powdered activated carbon, granular activated carbon, and pelletized activated carbon depending on the its particle size. Fine pores in the angstrom unit are present exist in the entire activated carbon, adsorbing not only solid molecules but also liquid and gas molecules. Its purpose is mainly to perform decolorization, deodorization and removal of impurities, and its application is diverse, including air pollution prevention and wastewater treatment.

The solubility of sophorolipid in water is relatively low compared to organic solvents, the sophorolipid is highly soluble in solvents such as methanol, ethanol, and ethyl acetic acid, but is dissolved in organic ethanol to use an eco-friendly solvent. At this time, it is desirable for the ethanol to have a purity of 95% or higher. The sophorolipid mixture and activated carbon are added to organic ethanol and then stirred.

The sophorolipid mixture may be treated at a ratio of 5 to 15 parts by weight based on 100 parts by weight of ethanol, and the activated carbon may be treated at a ratio of 5 to 15 parts by weight based on 100 parts by weight of ethanol. When the amount of the activated carbon treated is less than the above range, the effects such as decolorization and deodorization due to addition are minimal, and conversely, when the amount exceeds the above range, the effects such as discoloration and deodorization due to addition are no longer improved.

At this time, the activated carbon may include any shape, such as powdered, pelletized, granular, sheet-like, or fibrous activated carbon. Specifically, it is desirable to use powdered activated carbon to remove abnormal odor-causing substances and improve color and turbidity.

The oxidized and lactonic sophorolipids that have been treated with activated carbon are filtered to remove the activated carbon, and then vacuum concentration is used to completely remove the ethanol that dissolved the sophorolipids. Although an organic ethanol was used, residual ethanol may cause skin irritation when used as a cosmetic raw material. Ethanol removal is confirmed by gas chromatography (GC) analysis. A mixture of the oxidized and lactonic sophorolipids from which residual ethanol has been removed has a very high viscosity and shows fluidity at high temperatures, but shows almost no fluidity at room temperature.

### (3) Mixing the solution obtained after removing the activated carbon and ethanol with water to prepare an aqueous solution, and adjusting the pH of the aqueous solution to pH 5 or more and less than pH 7.

After adding water to improve viscosity, the fluidity of the sophorolipid mixture is secured by adjusting pH. The solubility of sophorolipids in water increases at pH 5.0 or higher, and sophorolipid molecules tend to decompose at pH 7.0 or higher. When sophorolipids are dissolved in water, the pH decreases, and based on this, when water is added to the sophorolipid mixture and the pH drops below 5.0, a base such as NaOH is continuously added to adjust the pH to between 5 and 7, preferably between pH 5 and 6. At this time, the solution obtained from operation (2) and water may be mixed at a weight ratio of 1:1 to 7:3.

Hereinafter, the content of the present disclosure will be described in detail through Examples and Test Examples. However, these are for explaining the present disclosure in more detail and the scope of the present disclosure is not limited thereto.

### Example 1. Separation and purification of oxidized and lactonic sophorolipids

The medium volume in the 5 L fermentor is 2 L, and fed-batch culture was performed using a culture fluid composed of 100.0 g of glucose, 5.0 g of non-animal malt extract, 1.0 g of KH₂PO₄, 0.5 g of MgSO₄·7H₂O, 0.1 g of CaCl₂·2H₂O, 0.1 g of NaCl, and 50.0 g of rapeseed oil per 1 L of medium. A seed culture fluid was prepared using a 1 L round Erlenmeyer flask including 200 ml of a culture fluid. Culture was stopped at the early exponential phase, when microbial strains were most actively proliferating, and these cells were recovered and used as the seed culture fluid to inoculate the fermentor. The culture conditions are maintained at a culture temperature of 25 °C and a dissolved oxygen (DO) concentration of 30%, the culture medium was prepared at pH 5.0, and when the pH decreased to 3.5 as the microorganisms grew, the pH was kept constant at 3.5 using 4N NaOH. When the concentrations of glucose and oil in the culture fluid decreased below a certain level, the glucose concentration was supplied intermittently to maintain 5 to 6% (w/v) and 2 to 3% (w/v) of rapeseed oil.

As a result of culturing the *Candida bombicola* strain under optimized culture conditions using the 5 L fermenter, a production concentration of 250 g/L and productivity of 0.69 g/L/h were confirmed through 360 hours of culture, and after standing still and separating the layers, the sophorolipid precipitate precipitated in the lower layer was recovered and subjected to separation, purification, and subsequent processes.

Membrane filtration, specifically ultrafiltration, is performed to remove impurities such as culture fluid compositions, proteins, and metabolites from the sophorolipid precipitate produced by culturing the genus Candida yeast fungus and to separate and purify a mixture of the oxidized and lactonic sophorolipids. In the case of proteins in the culture fluid, their molecular weight distribution varies, and proteins with relatively large molecular weights need to be removed through pretreatment to prevent membrane clogging during membrane filtration. Therefore, the primary purpose of ultrafiltration with a pore size corresponding to a molecular weight cutoff of 10,000 Da is to remove proteins and impurities with high molecular weight.

To confirm the sophorolipids separated by filtration, oxidized and lactonic sophorolipids were analyzed using high-performance liquid chromatography (HPLC). Finally, analysis samples were prepared by dissolving sophorolipids in 20% MeOH, and a column used in the sophorolipid HPLC analysis was Optimapak C18, the analysis conditions were a column temperature of 25 °C and a UV detector of 207 nm, acetonitrile (A) and water were used as mobile phases to provide a concentration gradient(0-15 minutes: 20% A; 15-40 minutes: 80% A; 40-50 minutes: 100% A; 50-70 minutes: 100% A; and 70-80 minutes: 20% A) over time, and the analysis was performed at a flow rate of 0.5 ml/min.

FIG. 1 is a graph showing the results of analysis of an oxidized sophorolipids and a lactonic sophorolipids using high-performance liquid chromatography (HPLC) to identify the sophorolipids separated after filtering the sophorolipid precipitate produced by culturing the genus Candida yeast fungus using an ultrafiltration membrane having a molecular weight cutoff of 10,000 Da.

Referring to FIG. 1, the peaks of a oxidized sophoroipids present between 33 minutes and 44 minutes and the peaks of a lactonic sophoroipids present between 49 minutes and 53 minutes were confirmed.

To separate and purify a mixture of the oxidized and lactonic sophorolipids from which relatively large molecular weight impurities were removed, membrane filtration was performed using an ultrafiltration membrane having a pore size corresponding to a molecular weight cutoff of 3,000 Da. The oxidized and lactonic sophorolipids that form micelles in aqueous solutions have a molecular weight of approximately 4,100 to 4,200 g/mol, and the molecular weight of impurities to be removed, such as culture fluid components and pigment substances, is expected to be mostly 1,000 g/mol or less. The membrane filtration results were concentrated, dried, recovered, and then subjected to HPLC analysis. As a result of membrane filtration, the mixture of the oxidized and lactonic sophorolipids concentrated on the outside of the membrane was recovered, concentrated in vacuum, dried, and then subjected to HPLC analysis. Referring to FIG. 2, it can be confirmed that the main components of the culture fluid, such as sugars and oils, were removed, and the content of the oxidized and lactonic sophorolipids increased.

The mixture of the oxidized and lactonic sophorolipids recovered through the membrane filtration has a turbid color and abnormal odor, and its high viscosity makes it difficult to use in cosmetic formulations. Therefore, treatment with activated carbon was performed for the purpose of improving the turbid color of the sophorolipid mixture and removing abnormal odor-causing substances. The activated carbon used is classified into granular activated carbon, pelletized activated carbon, and powdered activated carbon according to particle size, and more specifically, the effect on impurity removal varies depending on the pretreatment method and pore size.

Sophorolipids exhibit relatively high solubility in organic solvents compared to water, and the organic solvents includes methanol, ethanol, and ethyl acetic acid. In treatment with activated carbon, because sophorolipids need to be sufficiently dissolved in contact with activated carbon for activated carbon to perform its function, organic ethanol was used as a solvent for environmental friendliness. After dissolving 10 parts by weight of sophorolipids in ethanol relative to 100 parts by weight of ethanol, pelletized activated carbon, granular activated carbon, and powdered activated carbon in the same amount as the sophorolipids were added, respectively, and stirred. After stirring for 12 to 24 hours, the activated carbon was removed through filtration, and the recovered liquid was concentrated in vacuum and dried to remove ethanol from the sophorolipid mixture. Residual ethanol was analyzed using GC, and it was confirmed that it was not detected. Sensory tests were performed on the color and odor of the recovered sophorolipids, and the results are shown in FIG. 3.

Referring to Figure 3, the sophorolipid mixture treated with the powdered activated carbon had a reduced abnormal odor and almost no abnormal odor, and the turbid color was also improved from high turbidity and brown color to transparent amber. It can be confirmed that fluidity has also improved compared to before treatment with activated carbon. Referring to FIG. 4, a final recovery rate was 80% or more compared to the membranefiltered sophorolipid mixture.

The mixture of oxidized and lactonic sophorolipids separated and purified through membrane filtration and treatment with activated carbon had a high content and improved abnormal odor and turbid color, and high viscosity is still a matter in applications in various fields, including cosmetic formulations.

To improve this, an experiment was performed to improve the fluidity of the sophorolipid mixture by mixing water into the sophorolipid mixture and adjusting pH. Sophorolipids show relatively high solubility in water at pH 5.0 or more, and when sophorolipids are dissolved, the pH drops. Based on this, when sophorolipid and water were mixed in a weight ratio of 5:5, 6:4, or 7:3, and the pH fell below 5.0, 1N NaOH was added to maintain pH at 5.0 or more, more specifically pH 6.0, and at pH of 7.0 or more, sophorolipid molecules may break down.

HPLC analysis showed that when a high concentration of NaOH was added, an acetyl group in the sophorolipid molecule was removed and the molecule was broken, so that the NaOH concentration was fixed at 1 N. When the sophorolipid mixture and water were mixed without layer separation, pH adjustment and stirring were stopped, fluidity was checked, sensory tests for color and odor were performed, and HPLC analysis was also performed to confirm the content of the sophorolipid mixture. As a result of HPLC analysis, it was confirmed that the acetyl group was removed, and a content of lactonic sophorolipids was lowered, a content of oxidized sophorolipids increased slightly, and the ratio of oxidized and lactonic sophorolipids became 40:60 (FIG. 5). The fluidity of the high-viscosity sophorolipid mixture before mixing with water and adjusting pH was improved, showing a degree of flowability without staining the walls, and the color was diluted and became transparent when mixed with water (FIG. 6).

## Claims

1. A method for purifying sophorolipids, the method comprising:
(1) ultrafiltering a sophorolipid precipitate from a culture fluid of the genus Candida yeast fungus;
(2) adding ethanol and activated carbon to the ultrafiltration residue, adsorbing it, and then removing the activated carbon and ethanol; and
(3) mixing the solution obtained in operation (2) with water to prepare an aqueous solution, and adjusting the pH of the aqueous solution to pH 5 or more and less than pH 7.

2. The method of claim 1, wherein the ultrafiltering comprises the following:
filtering with an ultrafiltration membrane having a molecular weight cutoff of 10,000 Da; and
filtering with an ultrafiltration membrane having a molecular weight cutoff of 1,000 to 3,000 Da.

3. The method of claim 1, wherein the activated carbon is added in an amount of 5 to 15 parts by weight based on 100 parts by weight of ethanol.

4. The method of claim 1, wherein the pH of the aqueous solution is adjusted by adding sodium hydroxide (NaOH).
